(19) Europäisches Patentamt
European Patent Office
Office européen des brevets

(11) EP 0 614 893 B1

# (12) FASCICULE DE BREVET EUROPEEN

(45) Date de publication et mention de la délivrance du brevet:
**27.05.1998 Bulletin 1998/22**

(51) Int Cl.[6]: **C07D 265/36**, C07D 413/06, C07D 413/14, C07D 413/12, A61K 31/535

(21) Numéro de dépôt: **94400382.1**

(22) Date de dépôt: **23.02.1994**

(54) **Dérivés de benzoxazine, leur préparation, et leur application en thérapeutique**

Benzoxazinderivate, deren Herstellung und deren Verwendung in Therapie

Benzoxazine derivatives, their preparation and their use in therapy

(84) Etats contractants désignés:
**AT BE CH DE DK ES FR GB GR IE IT LI LU MC NL PT SE**

(30) Priorité: **08.03.1993 FR 9302659**

(43) Date de publication de la demande:
**14.09.1994 Bulletin 1994/37**

(73) Titulaire: **SYNTHELABO**
**92350 Le Plessis Robinson (FR)**

(72) Inventeurs:
- **Williams, Paul Howard**
  **F-75011 Paris (FR)**
- **Zard, Lydia**
  **F-91190 Gif sur Yvette (FR)**
- **Purcell, Thomas Andrew**
  **F-78490 Montfort l'Amaury (FR)**
- **Galtier, Daniel**
  **F-78280 Guyancourt (FR)**
- **Muller, Jean-Claude**
  **F-91390 Morsang sur Orge (FR)**
- **George, Pascal**
  **F-78730 St Arnoult en Yvelines (FR)**
- **Frost, Jonathan**
  **F-91320 Wissous (FR)**
- **Pasau, Patrick**
  **F-92220 Bagneux (FR)**
- **Rousselle, Corinne**
  **F-92260 Fontenay aux Roses (FR)**
- **Bartsch, Régine**
  **F-67800 Bischheim (FR)**

(74) Mandataire: **Ludwig, Jacques et al**
**SYNTHELABO,**
**Service Brevets,**
**B.P. 72**
**92352 Le Plessis Robinson Cédex (FR)**

(56) Documents cités:
**WO-A-89/07596** **DE-B- 1 287 584**

- **COMPTES RENDUS DES SEANCES DE L'ACADEMIE DES SCIENCES, SERIE C vol. 274, no. 11 , 1972 , PARIS pages 1081 - 1083 H. TECHER 'Tétrahydro-1.2.3.3a 4H-pyrrolo-(2.1-c)benzoxazine-1.4 et dérivés.'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

WO-A-8907596 divulgue des dérivés de 1,4-benzoxazine utiles en thérapie comme tranquilisants.

Les composés de l'invention répondent à la formule générale (I)

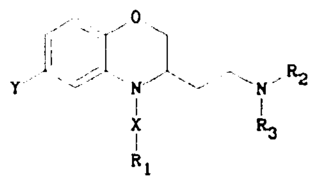

(I)

dans laquelle

Y représente un atome d'hydrogène, de fluor ou de chlore ou un groupe méthyle ou méthoxy,

$R_1$ représente soit un groupe phényle substitué par un atome de fluor ou par un groupe choisi parmi les groupes méthyle, méthoxy, trifluorométhyle et phényle, soit un groupe thién-2-yle,

$R_2$ représente un groupe méthyle,

$R_3$ représente soit un groupe ($C_1$-$C_4$)alkyle, soit un groupe phényl($C_1$-$C_2$)alkyle éventuellement substitué sur le noyau par 2 à 3 groupes méthoxy, soit un groupe 2-(pyridin-2-yl)éthyle, ou bien

$R_2$ et $R_3$ forment, avec l'azote, soit un groupe 4-phényl(pipéridin-1-yle), soit un groupe 4-phénylméthyl(pipéridin-1-yle), soit un groupe 1,2,3,4-tétrahydroisoquinoléin-2-yle, soit un groupe 6-méthoxy-1,2,3,4-tétrahydroisoquinoléin-2-yle, soit un groupe 5,8-diméthoxy-1,2,3,4-tétrahydroisoquinoléin-2-yle, soit un groupe 6,7-diméthoxy-1,2,3,4-tétrahydroisoquinoléin-2-yle, soit un groupe 2,3,4,5-tétrahydro-1*H*-3-benzazépin-3-yle, soit un groupe 7,8-diméthoxy-2,3,4,5-tétrahydro-1*H*-3-benzazépin-3-yle, et

X représente soit un groupe carbonyle, soit un groupe sulfonyle.

Les composés préférés sont ceux répondant à la formule générale (I) dans laquelle

$R_1$ représente un groupe phényle substitué en 3 par un groupe trifluorométhyle,

$R_2$ et $R_3$ forment, avec l'azote, un groupe 6,7-diméthoxy-1,2,3,4-tétrahydroisoquinoléin-2-yle, et

X représente un groupe carbonyle.

La molécule représentée par la formule générale (I) possédant un atome de carbone asymétrique, les composés de l'invention peuvent exister sous forme d'énantiomères purs ou de mélange d'énantiomères. Enfin, les composés de l'invention peuvent se présenter sous forme de bases libres ou de sels d'addition à des acides pharmaceutiquement acceptables.

Ces différentes formes font partie de l'invention.

Conformément à l'invention, on peut préparer les composés pour lesquels X représente un groupe carbonyle, selon le schéma 1 de la page suivante.

On fait réagir un 2-aminophénol de formule générale (II), dans laquelle Y est tel que défini ci-dessus, avec de l'anhydride trifluoracétique de formule (III), en présence d'une base telle que la pyridine, dans un solvant tel que l'éther. On obtient un amide de formule générale (IV) que l'on fait réagir avec du 4-bromobut-2-énoate d'éthyle, de formule (V) en présence d'une base telle que le méthanolate de sodium, dans un solvant tel que l'éthanol, à une température de l'ordre de 80°C. On réduit ensuite la fonction ester du dérivé de 3,4-dihydro-2*H*-1,4-benzoxazine-3-acétate d'éthyle, de formule générale (VI) avec un agent réducteur tel que l'hydrure de lithium et d'aluminium, pour obtenir le dérivé de 3,4-dihydro-2*H*-1,4-benzoxazine-3-éthanol de formule générale (VII), que l'on fait réagir, dans un solvant tel que le dichlorométhane, avec un chlorure d'acide de formule générale (VIII), dans laquelle $R_1$ est tel que défini ci-dessus, pour obtenir un alcool de formule générale (IX), que l'on fait réagir avec du chlorure de thionyle pour obtenir un composé de formule générale (X). Finalement, on fait réagir ce dernier avec une amine de formule générale (XI), dans laquelle $R_2$ et $R_3$ sont tels que définis ci-dessus, pour obtenir un composé de formule (I bis), qui correspond à la formule générale (I) où X représente un groupe carbonyle.

Schéma 1

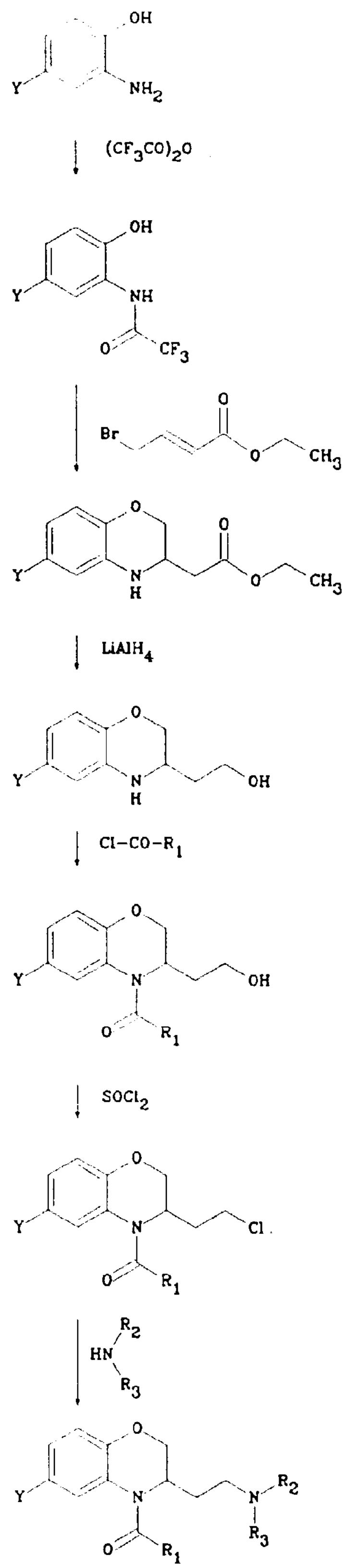


(II)

(III)

(IV)

(V)

(VI)

(VII)

(VIII)

(IX)

(X)

(XI)

(I bis)

Lorsque X représente un groupe sulfonyle, on peut préparer les composés de formule générale (I) selon le schéma 2 qui suit.

Schéma 2

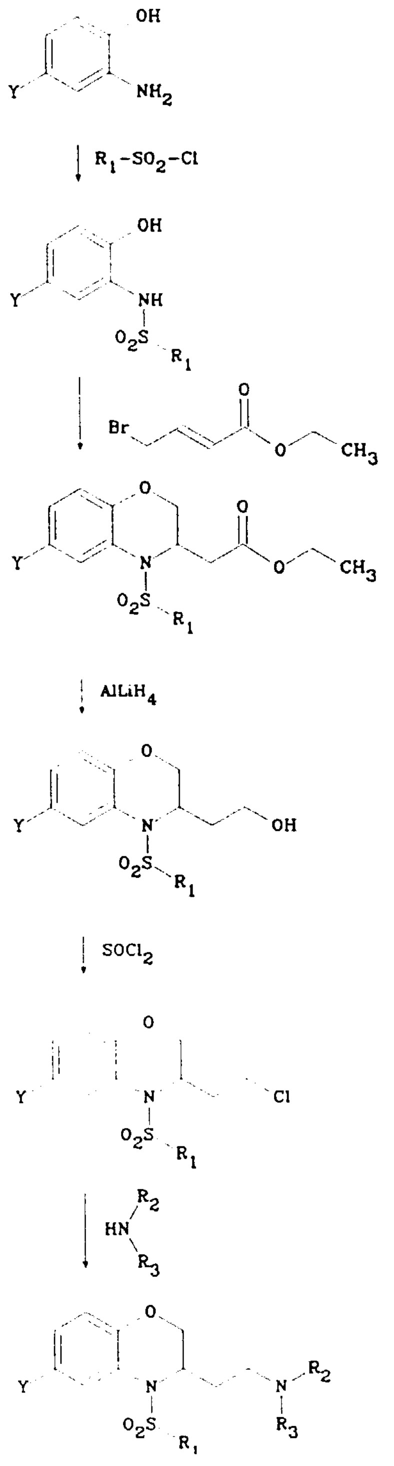


(II)

(III')

(IV')

(V)

(VI')

(VII')

(X')

(XI)

(I ter)

On fait réagir un 2-aminophénol de formule générale (II), dans laquelle Y est tel que défini ci-dessus, avec un chlorure de formule générale (III'), en présence d'une base telle que la pyridine. On obtient un composé de formule générale (IV') que l'on fait réagir avec du 4-bromobut-2-énoate d'éthyle de formule (V) en présence d'une base telle que le méthanolate de sodium, dans un solvant tel que l'éthanol, à une température de 80°C. On réduit ensuite la

fonction ester du composé de formule générale (VI') avec un agent réducteur tel que l'hydrure de lithium et d'aluminium pour obtenir le composé de formule générale (VII') que l'on fait réagir, dans un solvant tel que le chloroforme, avec du chlorure de thionyle, pour obtenir le composé de formule générale (X'), que l'on condense finalement avec une amine de formule générale (XI), dans laquelle $R_2$ et $R_3$ sont tels que définis ci-dessus.

Les produits de départ sont disponibles dans le commerce ou décrits dans la littérature, ou peuvent être synthétisés selon des méthodes qui y sont décrites ou qui sont connues de l'homme du métier. En particulier, le 2-amino-4-méthoxyphénol est décrit dans *J. Am. Chem. Soc.* (1949) **71** 1265.

Si on désire obtenir un composé de formule générale (I) optiquement pur, on peut utiliser un alcool de formule générale (IX) ou (VII') optiquement pur, que l'on aura isolé, par exemple, par une méthode enzymatique.

Le principe de base de cette méthode enzymatique consiste à séparer un alcool optiquement pur et l'acétate correspondant, de configuration opposée, par exemple par chromatographie sur colonne de gel de silice.

Selon une première variante, on soumet l'alcool de formule (IX) ou (VII') racémique à une acylation chimique, par exemple au moyen d'anhydride acétique, on hydrolyse de manière stéréospécifique, en présence d'une enzyme, l'un des deux énantiomères de l'acétate racémique, et on sépare l'acétate qui n'a pas été hydrolysé. On obtient un alcool optiquement pur et un acétate optiquement pur de configuration opposée, qui peut, si on le désire, être lui-même hydrolysé par voie chimique ou enzymatique pour fournir le second énantiomère de l'alcool.

Selon une seconde variante on soumet l'alcool de formule (IX) ou (VII') racémique à une acylation stéréospécifique en présence d'une enzyme qui catalyse l'estérification d'un seul des énantiomères, par exemple au moyen d'acétate de vinyle. Comme précédemment, on obtient un alcool optiquement pur et un acétate optiquement pur de configuration opposée, qui peut, si on le désire, être lui même hydrolysé par voie chimique ou enzymatique, pour fournir le second énantiomère de l'alcool.

Dans les deux variantes on peut, selon l'enzyme utilisée, obtenir l'énantiomère lévogyre ou dextrogyre de l'alcool (IX) ou (VII') et son acétate de configuration opposée.

Les enzymes utilisables sont par exemple les lipases de Mucor Miehei, de Penicillium cyclopium ou de germe de blé.

Les composés intermédiaires sont nouveaux et font également partie de l'invention. Ils répondent à la formule générale (XII)

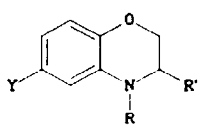

(XII)

dans laquelle Y est tel que défini ci-dessus et soit R représente un atome d'hydrogène et R' un groupe 2-hydroxyéthyle, soit R représente un groupe $-COR_1$ où $R_1$ est un groupe phényle substitué par un atome de fluor ou par un groupe méthyle, méthoxy, trifluorométhyle et phényle, ou un groupe thién-2-yle et R' représente un groupe 2-hydroxyéthyle ou un groupe 2-chloroéthyle, soit R représente un groupe $-SO_2R_1$ où $R_1$ est un groupe phényle substitué par un atome de fluor ou par un groupe méthyle, méthoxy, trifluorométhyle et phényle, ou un groupe thién-2-yle et R' représente un groupe 2-hydroxyéthyle ou un groupe 2-chloroéthyle ou un groupe éthoxycarbonylméthyle.

Les exemples qui suivent illustrent en détail la préparation de quelques composés selon l'invention. Les microanalyses élémentaires et les spectres IR et RMN confirment les structures des produits obtenus.

Les numéros indiqués entre parenthèses dans les titres des exemples correspondent à ceux du tableau donné plus loin.

Exemple 1 (Composé N°28)

(±) 3-[2-(7,8-diméthoxy-2,3,4,5-tétrahydro-1*H*-3-benzazépin-3-yl)éthyl]-4-[3-(trifluorométhyl)benzoyl]-3,4-dihydro-2*H*-1,4-benzoxazine, fumarate.

1.1. *N*-(2-hydroxyphényl)trifluoroacétamide.

Dans un réacteur de 4 litres, sous agitation magnétique, on met en suspension dans 1,5 litres d'éther, 104 g (0,95 mole) de 2-aminophénol auxquels on ajoute 77 ml de pyridine. On refroidit le milieu réactionnel par un mélange de glace et d'éthanol. On ajoute, goutte à goutte, en 1 heure, 200 g (0,95 mole) d'anhydride trifluoracétique. On laisse revenir à la température ambiante puis on continue l'agitation pendant 1 heure. On ajoute au milieu réactionnel de l'eau glacée, on décante, on lave la phase organique successivement par 1 litre d'une solution 1 N d'acide chlorhydrique, par de l'eau, par une solution saturée d'hydrogénocarbonate de sodium puis par une solution saturée de chlorure de sodium. On sèche sur sulfate de magnésium et on évapore à sec. On obtient 170 g de produit que l'on utilise tel quel dans l'étape suivante.

1.2. (±)-3,4-dihydro-2*H*-1,4-benzoxazine-3-acétate d'éthyle.

Dans un réacteur de 4 litres, sous agitation magnétique, on place 164 g (0,8 mole) du composé obtenu en 1.1 en solution dans 2 litres d'éthanol. On ajoute successivement 151 ml d'une solution de méthanolate de sodium 5,3 N et 154,43 g (0,8 mole) de 4-bromobut-2-énoate d'éthyle. On chauffe à 80°C pendant 1 heure. On évapore à sec, on reprend par 500 ml d'eau et 200 ml de soude 1 N puis on extrait par de l'éther. On lave la phase organique par une solution saturée de chlorure de sodium, on sèche sur du sulfate de magnésium et on évapore à sec. On récupère 153 g de produit que l'on purifie par chromatographie sur colonne de gel de silice, en éluant par du dichlorométhane.

On obtient 55 g de produit.

1.3. (±)-3,4-dihydro-2*H*-1,4-benzoxazine-3-éthanol.

Dans un réacteur de 2 litres, on place 300 ml de tétrahydrofurane et on refroidit par un mélange de glace et de sel. Sous courant d'argon, on ajoute 15 g d'hydrure de lithium et d'aluminium, puis goutte à goutte, 55 g (0,25 mole) du composé obtenu en 1.2 en solution dans 300 ml de tétrahydrofurane. On laisse agiter pendant 2 heures. On refroidit le réacteur par un mélange de neige carbonique et d'acétone, puis on ajoute, goutte à goutte 50 ml d'eau et 20 ml d'une solution de soude 1 N. On laisse sous agitation pendant 2 heures et une nuit au repos. On filtre le précipité sur kieselguhr, on le rince successivement avec du tétrahydrofurane et de l'acétate d'éthyle et on concentre à sec. On isole 40 g de produit brut que l'on purifie par chromatographie sur colonne de gel de silice en éluant avec un mélange hexane/acétate d'éthyle (50/50).

On obtient 35 g de produit.

1.4. (±)-4-[3-(trifluorométhyl)benzoyl]-3,4-dihydro-2*H*-1,4-benzoxazine-3-éthanol.

Dans un ballon de 100 ml, on place 25 ml de dichlorométhane, 8,96 g (0,08 mole) du composé obtenu en 1.3, 7,6 g (0,055 mole) de carbonate de potassium et on ajoute, goutte à goutte, 8,3 ml (0,055 mole) de chlorure de 3-(trifluorométhyl)benzoyle en solution dans 25 ml de dichlorométhane. On laisse à la température ambiante, sous agitation magnétique, pendant 2 heures. On récupère la phase organique, on la lave successivement par une solution de soude 1 N, par de l'eau puis par une solution saturée de chlorure de sodium. On la sèche sur sulfate de magnésium et on la concentre à sec. On purifie l'huile obtenue par chromatographie sur colonne de gel de silice en éluant par un mélange hexane/acétate d'éthyle (50/50). On isole 11 g d'huile jaune qui cristallise après une nuit au repos. On purifie 2 g de cette huile par chromatographie sur colonne de gel de silice en éluant par un mélange hexane/acétate d'éthyle (3/2).

On obtient 1,3 g de produit.

1.5. (±)-3-(2-chloroéthyl)-4-[3-(trifluorométhyl)benzoyl]3,4-dihydro-2*H*-1,4-benzoxazine.

A 3,51 g (0,01 mole) du composé obtenu en 1.4 en solution dans 50 ml de dichlorométhane, on ajoute 2,9 ml de chlorure de thionyle et on laisse, sous agitation, à la température ambiante, pendant 3 heures. On ajoute de nouveau 2,9 ml de chlorure de thionyle et on laisse, sous agitation, à la température ambiante, pendant 2 heures. On concentre à sec, on reprend par du toluène et on concentre de nouveau à sec. On obtient 3,2 g de produit.

1.6. chlorure de 3,4-diméthoxybenzènacétyle.

A 95 g (0,48 mole) d'acide 3,4-diméthoxybenzènacétique en solution dans 200 ml de dichlorométhane, on ajoute 103,7 ml (1,42 moles) de chlorure de thionyle. On laisse, sous agitation, à la température ambiante, pendant 18 heures. On évapore les solvants.

On isole 1,4 g de produit brut sous forme d'une huile brunâtre.

1.7. *N*-(2,2-diméthoxyéthyl)-3,4-diméthoxybenzènacétamide.

A une solution de 52,7 ml (0,48 mole) de 2,2-diméthoxyéthanamine, refroidie à 10°C, et contenant 67,5 ml de triéthylamine dans 500 ml de dichlorométhane, on ajoute, goutte à goutte, 104 g (0,48 mole) du composé obtenu en 1.6 en solution dans 250 ml de dichlorométhane. Lorsque l'addition est terminée, on laisse revenir à la température ambiante et on laisse sous agitation pendant 1 heure. On ajoute 500 ml d'eau glacée et on décante la phase organique. On la récupère, on la lave par une solution saturée de sulfate de magnésium et on la concentre à sec. On obtient 128 g de produit sous forme d'une huile visqueuse.

1.8. 7,8-diméthoxy-1,3-dihydro-2*H*-3-benzazépin-2-one.

On laisse, sous agitation, pendant 8 heures, à la température ambiante, 128 g (0,45 mole) du composé obtenu en 1.7 en solution dans un mélange de 640 ml d'acide chlorhydrique concentré et de 640 ml d'acide acétique. On laisse, sous agitation, pendant 3 jours à la température ambiante. On ajoute 2 kg de glace et on isole, par filtration, le produit obtenu qui a précipité dans le milieu, on le rince par un mélange eau/méthanol et on le sèche à l'étuve.

On obtient 42 g de produit.
Point de fusion : 240-244°C.

1.9. 7,8-diméthoxy-1,3,4,5-tétrahydro-2*H*-3-benzazépin-2-one.

On réalise l'hydrogénation du composé obtenu en 1.8, sous une pression de 0,42 MPa à 50°C, en présence de 1 g de palladium sur charbon à 10%, pendant 3 heures. On concentre à sec, on filtre sur kieselguhr et on rince avec de l'acide acétique. On reprend le résidu par du dichlorométhane et on le lave successivement par une solution saturée d'hydrogénocarbonate de sodium puis par de l'eau. On le sèche sur sulfate de magnésium et on concentre à sec.
On obtient 13,2 g de produit.
Point de fusion : 186-190°C.

1.10. 7,8-diméthoxy-1,3,4,5-tétrahydro-2*H*-3-benzazépine, chlorhydrate.

A une suspension de 2,2 g (0,01 mole) du composé obtenu en 1.9, en solution dans 25 ml de tétrahydrofurane sec, sous argon, on ajoute au goutte à goutte, à la température ambiante, 20 ml d'une solution 1 M de diborane dans du tétrahydrofurane. On chauffe à la température de reflux pendant 2 heures. On refroidit par un mélange de glace et d'alcool et on ajoute, goutte à goutte, 30 ml d'acide chlorhydrique 6 N. On chauffe, pendant 1 heure, à 80°C. On alcalinise par une solution de soude 4 N et on extrait par de l'acétate d'éthyle. On recueille la phase organique, on la lave par une solution saturée de chlorure de sodium, on sèche sur sulfate de magnésium et on concentre à sec. On reprend le résidu dans 100 ml de propan-2-ol chlorhydrique 0,1 N et on isole le précipité formé par filtration. On le sèche et on obtient 1 g de produit.
Point de fusion : 236°C.

1.11. (±)-3-[2-(7,8-diméthoxy-2,3,4,5-tétrahydro-1*H*-3-benzazepin-3-yl)éthyl]-4-[3-(trifluorométhyl)benzoyl]-3,4-dihydro-2*H*-1,4-benzoxazine, fumarate.

On mélange 3 g (0,015 mole) du composé obtenu en 1.5, 3,56 g (0,0096 mole) du composé obtenu en 1.10, 2,66 g de carbonate de potassium, 100 mg d'iodure de potassium et 50 ml de diméthylformamide. On chauffe le mélange à 80°C pendant 4 heures puis on le jette sur un mélange de glace et d'eau. On extrait à l'éther, on lave la phase organique par une solution saturée de chlorure de sodium, on sèche sur sulfate de magnésium et on concentre à sec. On purifie l'huile résiduelle par chromatographie sur colonne de gel de silice en éluant par un mélange dichlorométhane/méthanol (99/1), puis par un mélange dichlorométhane/méthanol (98/2). On obtient 1,8 g de base. On prépare le fumarate en ajoutant un équivalent d'acide fumarique. On l'isole et on le recristallise dans le propan-2-ol.
Point de fusion : 182-184°C.

<u>Exemple 2</u> (Composé N°16)

(±)-3-[2-(6,7-diméthoxy-1,2,3,4-tétrahydroisoquinoléin-2-yl)éthyl]-4-[(4-méthylphényl)sulfonyl]-3,4-dihydro-2*H*-1,4-benzoxazine, oxalate.

2.1. *N*-(2-hydroxyphényl)-4-méthylbenzènesulfonamide.

A 20 g (0,18 mole) de 2-aminophénol, on ajoute 35 g (0,18 mole) de chlorure de tosyle et 60 ml de pyridine. On laisse, sous agitation, à la température ambiante pendant une nuit. On ajoute de l'eau et on extrait 2 fois par de l'éther. On lave les phases organiques successivement avec de l'eau, de l'acide chlorhydrique 1 N et de nouveau 2 fois avec de l'eau. On sèche sur du sulfate de magnésium et on concentre à sec.
On obtient 44 g de produit.

2.2. (±)-4-[(4-méthylphényl)sulfonyl]-3,4-dihydro-2*H*-1,4-benzoxazine-3-acétate d'éthyle

A 5,3 g (0,02 mole) du composé obtenu en 2.1, on ajoute 5,1 g (0,03 mole) de 4-bromobut-2-énoate d'éthyle, 3,8 ml d'une solution de méthanolate de sodium 5,3 N et 25 ml d'éthanol. On chauffe le mélange à la température de reflux pendant une nuit. On évapore à sec, on reprend le résidu successivement par de l'acétate d'éthyle, par de l'eau et finalement par une solution de soude 1 N. On recueille la phase organique, on l'extrait et on la lave avec de l'eau puis avec une solution saturée en chlorure de sodium. On la sèche sur du sulfate de magnésium puis on évapore à sec.
On obtient 8 g de produit que l'on utilise tel quel dans l'étape suivante.

2.3. (±)-4-[(4-méthylphényl)sulfonyl]-3,4-dihydro-2*H*-1,4-benzoxazine-3-éthanol.
On met en solution, sous argon, 0,23 g (0,006 mole) d'hydrure de lithium et d'aluminium dans 20 ml de tétrahydrofurane. On ajoute goutte à goutte 1,5 g (0,004 mole) du composé obtenu en 2.2 en solution dans 5 ml de tétrahydrofurane. Lorsque la réaction est terminée, on ajoute successivement 0,7 g d'eau, 0,3 g d'une solution de soude 1 N et de nouveau 0,7 g d'eau. On filtre le mélange sur kieselguhr, on lave avec du tétrahydrofurane et on évapore. On reprend le résidu par de l'acétate d'éthyle et on lave la phase organique avec de l'eau. On sèche et on évapore à sec.
On obtient 1,4 g de produit.

2.4. (±)-3-(2-chloroéthyl)-4-[(4-méthylphényl)sulfonyl]-3,4-dihydro-2*H*-1,4-benzoxazine.
On dissout 2 g (0,006 mole) de l'alcool obtenu en 2.3 dans 15 ml de chloroforme. On ajoute goutte à goutte 2,2 g (0,018 mole) de chlorure de thionyle et une goutte de diméthylformamide. On chauffe à la température de reflux, pendant 5 heures. On évapore à sec, on reprend le résidu par un volume minimum de toluène et on évapore de nouveau à sec. On obtient 2,1 g de produit.

2.5. (±)-3-[2-(6,7-diméthoxy-1,2,3,4-tétrahydroisoquinoléin-2-yl)éthyl]-4-[(4-méthylphényl)sulfonyl]-3,4-dihydro-2*H*-1,4-benzoxazine, oxalate.
A 1,2 g (0,0034 mole) du composé obtenu en 2.4, on ajoute 0,65 g (0,0034 mole) de 6,7-diméthoxy-1,2,3,4-tétrahydroisoquinoléine et 6 ml de 3-méthylbutanol. On chauffe le mélange à 80 °C, pendant une nuit et on évapore à sec. On reprend le résidu par de l'ammoniaque diluée et on extrait deux fois avec de l'éther. On lave les phases éthérées par de l'eau, on les sèche sur sulfate de magnésium et on évapore à sec. On purifie le produit obtenu par chromatographie sur colonne de gel de silice en éluant par un mélange de dichlorométhane/méthanol à 1 %.
On obtient 0,8 g de produit.
On prépare l'oxalate en ajoutant un équivalent d'acide oxalique. On le recristallise dans un mélange acétate d'éthyle/éthanol.
On obtient 0,6 g d'oxalate.
Point de fusion : 190-192°C

Exemple 3 (Composé N°18)

(±)-3-[2-(6,7-diméthoxy-1,2,3,4-tétrahydroisoquinoléin-2-yl)éthyl]-4-[3-(trifluorométhyl)benzoyl]-3,4-dihydro-2*H*-1,4-benzoxazine, oxalate.

3.1. (±)-3-[2-(6,7-diméthoxy-1,2,3,4-tétrahydroisoquinoléin-2-yl)éthyl]-4-[3-(trifluorométhyl)benzoyl]-3,4-dihydro-2*H*-1,4-benzoxazine, oxalate.
A une solution de 2,0 g (0,005 mole) du composé obtenu en 1.5, en solution dans 10 ml de diméthylformamide, à la température ambiante, sous agitation et sous atmosphère d'argon, on ajoute 1,24 g (0,005 mole) de chlorhydrate de thoxy-1,2,3,4-tétrahydroisoquinoléine, 4 g de carbonate de potassium et 0,9 g d'iodure de potassium. On chauffe le mélange à 80°C pendant 4 heures. On le refroidit et on ajoute 40 ml d'eau et 100 ml d'éther. On sépare les phases et on extrait la phase aqueuse avec 2 fois 100 ml d'éther. On rassemble les phases organiques, on les lave par 100 ml d'une solution saturée de chlorure de sodium, on les sèche sur sulfate de magnésium, on filtre et on évapore à sec. On obtient 3 g de produit sous forme d'une huile brune que l'on purifie par chromatographie sur colonne de gel de silice en éluant par un mélange méthanol/dichlorométhane (1/9). On obtient 1,89 g de base sous la forme d'une huile jaune. On prépare l'oxalate en ajoutant un équivalent d'acide oxalique. On l'isole et on le recristallise sous forme de cristaux blancs, dans un mélange isopropanol/éther isopropylique.
Point de fusion : 126-128°C.

Exemple 4 (Composé N°18a)

(+)-3-[2-(6,7-diméthoxy-1,2,3,4-tétrahydroisoquinoléin-2-yl)éthyl]-4-[3-(trifluorométhyl)benzoyl]-3,4-dihydro-2*H*-1,4-benzoxazine, oxalate.

4.1. (+)-4-[3-(trifluorométhyl)benzoyl]-3,4-dihydro-2*H*-1,4-benzoxazine-3-éthanol.
On met en suspension, 8,7 g (0,025 mole) d'alcool racémique obtenu en 1.4 dans 1,09 litres d'hexane. On ajoute 7,6 ml (0,082 mole) d'acétate de vinyle et 4,35 g de lipase de Mucor Miehei. On laisse pendant 15 heures à la température ambiante et on filtre le mélange sous vide. On obtient 10,7 g d'huile jaune qui contient un mélange d'alcool dextrogyre et d'acétate lévogyre. On les sépare par chromatographie sur colonne de gel de silice en éluant par un mélange acétate d'éthyle/cyclohexane (1/1).
On obtient 3,62 g d'alcool dextrogyre chimiquement pur.
Pouvoir rotatoire : $[\alpha]_D^{20}$ + 62° (c = 0,99 ; dichlorométhane) Excès énantiomérique : ee=99,7% (CLHP chirale)

4.2. (+)-3-(2-chloroéthyl)-4-[3-(trifluorométhyl)benzoyl]-3,4-dihydro-2*H*-1,4-benzoxazine.

A une solution de 4 g ( 0,011 mole) de l'alcool obtenu en 4.1, dans 20 ml de dichlorométhane, à la température ambiante, sous agitation et sous atmosphère d'argon, on ajoute 3 ml (0,041 mole) de chlorure de thionyle. On continue l'agitation à la température ambiante pendant 18 heures. On évapore à sec et on utilise le produit obtenu tel quel dans l'étape suivante.

On obtient 4,46 g de produit.

4.3. (+)-3-[2-(6,7-diméthoxy-1,2,3,4-tétrahydroisoquinoléin-2-yl)éthyl]-4-[3-(trifluorométhyl)benzoyl]-3,4-dihydro-2*H*-1,4-benzoxazine, oxalate.

A une solution de 4,46 g (0,012 mole) du composé obtenu en 4.2 dans 40 ml de diméthylformamide, à la température ambiante, sous agitation et sous atmosphère d'argon, on ajoute 2,31 g (0,012 mole) de chlorhydrate de 6,7-diméthoxy-1,2,3,4-tétrahydroisoquinoléine et 3,31 g (0,024 mole) de carbonate de potassium. On chauffe le mélange pendant 4 heures à 80°C puis on le refroidit. On ajoute successivement 40 ml d'eau et 100 ml d'éther, on sépare les phases et on extrait la phase aqueuse avec deux fois 100 ml d'éther. On rassemble les phases organiques et on les lave par 100 ml d'une solution saturée de chlorure de sodium. On les sèche sur sulfate de magnésium, on filtre et on évapore à sec. On obtient 7 g de produit que l'on purifie par chromatographie sur colonne de gel de silice en éluant par un mélange méthanol/dichlorométhane (1/9).

On obtient 1,17 g de base sous la forme d'une huile. On prépare l'oxalate en ajoutant un équivalent d'acide oxalique. On l'isole et on le recristallise, sous forme de cristaux blancs, dans un mélange d'acétate d'éthyle, d'éther isopropylique et d'acétone.

Point de fusion : 128-129°C.

Pouvoir rotatoire : $[\alpha]_D^{20}$ + 69° (c = 0,976 ; méthanol).

<u>Exemple 5</u> (Composé N°18b)

(-)-3-[2-(6,7-diméthoxy-1,2,3,4-tétrahydroisoquinoléin-2-yl)éthyl]-4-[3-(trifluorométhyl)benzoyl]-3,4-dihydro-2*H*-1,4-benzoxazine, oxalate.

5.1. (-)-4-[3-(trifluorométhyl)benzoyl]-3,4-dihydro-2*H*-1,4-benzoxazine-3-éthanol.

On met en suspension, 8,7 g (0,025 mole) d'alcool racémique obtenu en 1.4 dans 1,09 litres d'hexane. On ajoute 7,6 ml (0,082 mole) d'acétate de vinyle et 4,35 g de lipase de Mucor Miehei. On laisse pendant 15 heures à la température ambiante et on filtre le mélange sous vide. On obtient 10,7 g d'huile jaune qui contient un mélange d'alcool dextrogyre et d'acétate lévogyre. On les sépare par chromatographie sur colonne de gel de silice en éluant par un mélange acétate d'éthyle/cyclohexane (1/1).

On obtient 6,14 g d'acétate lévogyre (ee = 70 %) sous forme d'huile que l'on triture dans 200 ml d'hexane. On obtient un précipité correspondant à l'acétate racémique que l'on filtre. On évapore le filtrat à sec.

On obtient 3,94 g d'acétate lévogyre chimiquement pur.

Pouvoir rotatoire : $[\alpha]_D^{20}$ = -43° (c = 1,2 ; dichlorométhane) Excès énantiomérique : ee=99,5 % (CLHP chirale).

On dissout 3,94 g d'acétate lévogyre dans 40 ml de toluène. On ajoute 200 ml de tampon phosphate ($KH_2PO_4$/$Na_2PO_4$) 0,01 M à pH 7,2 et 1,2 g de lipase de Mucor Miehei. On agite le mélange une nuit à la température ambiante en maintenant le pH constant par addition d'une solution aqueuse de soude 0,5 M à l'aide d'un pH-stat. On ajoute 100 ml d'éther éthylique, on sépare la phase organique et on extrait la phase aqueuse par deux fois 100 ml d'éther. On rassemble les phases organiques et on les lave avec une solution saturée de chlorure de sodium. On les sèche sur du sulfate de magnésium, on filtre et on évapore à sec. On obtient 2,8 g d'alcool lévogyre chimiquement pur.

Pouvoir rotatoire : $[\alpha]_D^{20}$ =-59,8° (c = 1,32 ; dichlorométhane)

Excès énantiomérique : ee=96,5 % (CLHP chirale).

5.2. (-)-3-(2-chloroéthyl)-4-[3-(trifluorométhyl)benzoyl]-3,4-dihydro-2*H*-1,4-benzoxazine.

A une solution de 2,8 g ( 0,080 mole) de l'alcool obtenu en 5.1, dans 20 ml de dichlorométhane, à la température ambiante, sous agitation et sous atmosphère d'argon, on ajoute 3 ml (0,041 mole) de chlorure de thionyle. On continue l'agitation à la température ambiante pendant 18 heures. On évapore à sec et on utilise le produit obtenu tel quel dans l'étape suivante.

On obtient 3,04 g de produit.

5.3. (-)-3-[2-(6,7-diméthoxy-1,2,3,4-tétrahydroisoquinoléin2-yl)éthyl]-4-[3-(trifluorométhyl)benzoyl]-3,4-dihydro-2*H*-1,4-benzoxazine, oxalate.

A une solution de 3,04 g (0,008 mole) du composé obtenu en 5.2 dans 40 ml de diméthylformamide, à la

température ambiante, sous agitation et sous atmosphère d'argon, on ajoute 3,17 g (0,016 mole) de chlorhydrate de 6,7-diméthoxy-1,2,3,4-tétrahydroisoquinoléine et 2,26 g (0,016 mole) de carbonate de potassium. On chauffe le mélange pendant 4 heures à 80 °C puis on le refroidit. On ajoute successivement 40 ml d'eau et 100 ml d'éther, on sépare les phases et on extrait la phase aqueuse avec deux fois 100 ml d'éther. On rassemble les phases organiques et on les lave par 100 ml d'une solution saturée en chlorure de sodium. On les sèche sur du sulfate de magnésium, on filtre et on évapore à sec. On obtient 5 g de produit que l'on purifie par chromatographie sur colonne de gel de silice en éluant par un mélange méthanol/dichlorométhane (1/9).
On obtient 1,30 g de base sous la forme d'une huile.
On prépare l'oxalate en ajoutant un équivalent d'acide oxalique. On l'isole et on le recristallise, sous forme de cristaux blancs, dans un mélange de propan-2-ol, d'éther diisopropylique et d'acétone.
Point de fusion : 129-130°C.
Pouvoir rotatoire : $[\alpha]_D^{20}$ = -71° (c = 1,03 ; méthanol).

Exemple 6 (Composé N°38)

(±)-3-[2-(6,7-diméthoxy-1,2,3,4-tétrahydroisoquinoléin-2-yl)éthyl]-6-méthyl-4-[3-(trifluorométhyl)benzoyl]-3,4-dihydro-2H-1,4-benzoxazine, oxalate.

6.1. *N*-(2-hydroxy-5-méthylphényl)trifluoroacétamide.

Dans un réacteur de 1 litre, sous agitation magnétique, on prépare une suspension de 350 ml d'éther diéthylique et 25 g (0,2 mole) de 2-amino-4-méthylphénol, on ajoute 20,5 ml de pyridine, on refroidit le milieu réactionnel par un mélange de glace et d'éthanol et on ajoute, goutte à goutte, en 1 heure, 28 ml (0,2 mole) d'anhydride trifluoroacétique. On laisse le mélange revenir à température ambiante et on maintient l'agitation pendant 2 heures. On ajoute de l'eau glacée, on sépare la phase organique, on la lave successivement avec de l'acide chlorhydrique 1 N, avec de l'eau, avec une solution saturée d'hydrogénocarbonate de sodium et avec une solution saturée de chlorure de sodium, on la sèche sur sulfate de magnésium et on évapore le solvant. On obtient 37,07 g de produit qu'on utilise tel quel dans l'étape suivante.

6.2. (±)-6-méthyl-3,4-dihydro-2*H*-1,4-benzoxazine-3-acétate d'éthyle.
Dans un réacteur de 3 litres, sous agitation magnétique et refroidi à 0°C, on introduit 760 ml d'éthanol et on ajoute lentement, par petites portions, 5,79 g (0,252 mole) de sodium puis, goutte à goutte, 37,45 (0,17 mole) de *N*-(2-hydroxy-5-méthylphényl)trifluoroacétamide et 43,7 g (0,17 mole) de 4-bromobut-2-énoate d'éthyle pur à 75%, et on chauffe le mélange à 110°C pendant 2 heures.
On évapore le solvant, on reprend le résidu avec 100 ml d'eau et 40 ml de soude 1 N, et on l'extrait avec de l'éther diéthylique. On lave la phase organique avec une solution saturée de chlorure de sodium, on la sèche sur sulfate de magnésium et on évapore le solvant. On obtient 28,58 g de produit qu'on purifie par chromatographie sur colonne de gel de silice en éluant avec un mélange cyclohexane/éther isopropylique (50/50).
On obtient 23,48 g de produit.

6.3. (±)-6-méthyl-3,4-dihydro-2*H*-1,4-benzoxazine-3-éthanol.

Dans un réacteur de 1 litre on place 150 ml de tétrahydrofurane, on le refroidit avec un mélange de glace et de sel et, sous atmosphère d'argon, on ajoute 6 g (0,158 mole) d'hydrure de lithium et d'aluminium puis, goutte à goutte, 23,48 g (0,099 mole) de (±)-6-méthyl-3,4-dihydro-2*H*-1,4-benzoxazine-3-acétate d'éthyle en solution dans 150 ml de tétrahydrofurane, et on maintient l'agitation pendant 1,5 heure. On refroidit le réacteur avec un mélange de neige carbonique et d'acétone, on ajoute, goutte à goutte, 40 ml d'eau et 20 ml de soude 1 N, et on laisse sous agitation pendant 0,5 heure.
On filtre le précipité sur kieselguhr, on le rince avec du tétrahydrofurane puis avec de l'acétate d'éthyle et on évapore le solvant. On isole 20,29 g de produit brut qu'on purifie par chromatographie sur colonne de gel de silice en éluant avec un mélange cyclohexane/acétate d'éthyle (50/50). On obtient 22,94 g de produit.

6.4. (±)-6-méthyl-4-[3-(trifluorométhyl)benzoyl]-3,4-dihydro-2*H*-1,4-benzoxazine-3-éthanol.

Dans un ballon de 1 litre on place 200 ml de dichlorométhane, 22,87 g (0,118 mole) de 6-méthyl-3,4-dihydro-2*H*-1,4-benzoxazine-3-éthanol, 17,53 g (0,125 mole) de carbonate de potassium et on ajoute, goutte à goutte, 26,19 g (0,125 mole) de chlorure de 3-(trifluorométhyl)benzoyle en solution dans 200 ml de dichlorométhane, et on maintient l'agitation à température ambiante pendant 3 heures.
On ajoute 120 ml de soude 1 N, on sépare la phase organique, on la lave à l'eau puis avec une solution saturée de chlorure de sodium, on la sèche sur sulfate de magnésium, et on purifie les 46,25 g d'huile obtenue par chromatographie sur colonne de gel de silice en éluant avec un mélange cyclohexane/acétate d'éthyle (6/4).

On obtient 19,66 g de produit.

6.5. (±)-3-(2-chloroéthyl)-6-méthyl-4-[3-(trifluorométhyl)benzoyl]-3,4-dihydro-2*H*-1,4-benzoxazine.

A 19,66 g (0,054 mole) de (±)-6-méthyl-4-[3-(trifluorométhyl)benzoyl]-3,4-dihydro-2*H*-1,4-benzoxazine-3-éthanol en solution) dans 230 ml de dichlorométhane on ajoute 19,6 ml (0,27 mole) de chlorure de thionyle et on agite le mélange à température ambiante pendant 4 heures.
On évapore le solvant et l'excès de chlorure de thionyle, on reprend le résidu avec du toluène, on l'évapore, et on purifie le résidu par chromatographie sur colonne de gel de silice en éluant avec un mélange cyclohexane/éther isopropylique (2/1).
On obtient 13,83 g de produit.

6.6. (±)-3-[2-(6,7-diméthoxy-1,2,3,4-tétrahydroisoquinoléin-2-yl)éthyl]-6-méthyl-4-[3-(trifluorométhyl)benzoyl]-3,4-dihydro-2*H*-1,4-benzoxazine, oxalate.

A une solution de 2 g (0,005 mole) de (±)-3-(2-chloroéthyl)-6-méthyl-4-[3-(trifluorométhyl)benzoyl]-3,4-dihydro-2*H*-1,4-benzoxazine dans 20 ml de *N,N*-diméthylformamide, à température ambiante, sous atmosphère d'argon et sous agitation magnétique, on ajoute 1,19 g (0,005 mole) de chlorhydrate de 6,7-diméthoxy-1,2,3,4-tétrahydroisoquinoléine, 1,78 g (0,013 mole) de carbonate de potassium et 0,82 g (0,005 mole) d'iodure de potassium, et on chauffe le mélange à 150°C pendant 1 heure.
On le refroidit, on ajoute 55 ml d'eau et 50 ml d'éther diéthylique, on sépare les phases, on extrait la phase aqueuse avec deux fois 50 ml d'éther diéthylique, on rassemble les phases organiques, on les lave avec 100 ml de solution saturée de chlorure de sodium, on les sèche sur sulfate de magnésium, on filtre et on évapore le solvant. On obtient 3,75 g de produit sous forme d'huile qu'on purifie par chromatographie sur colonne de gel de silice en éluant avec un mélange dichlorométhane/méthanol (95/5).
On obtient 0,450 g de base pure sous forme d'huile jaune. On prépare l'oxalate en ajoutant un équivalent d'acide oxalique, on l'isole et on le recristallise, sous forme de cristaux blancs, dans le propan-2-ol.
On obtient 0,180 g d'oxalate (rapport acide:base = 0,8:1).
Point de fusion : 164-166°C.

<u>Exemple 7</u> (Composé N°35)

(±) 6-chloro-3-[2-(2,3,4,5-tétrahydro-1*H*-3-benzazépin-3-yl)éthyl]-4-[3-(trifluorométhyl)benzoyl]-3,4-dihydro-2*H*-1,4-benzoxazine, oxalate.

7.1. *N*-(5-chloro-2-hydroxyphényl)trifluoroacétamide.

Dans un réacteur de 1 litre, sous agitation magnétique, on met en suspension, dans 320 ml d'éther diéthylique, 25 g (0,174 mole) de 2-amino-4-chlorophénol, on ajoute 18 ml de pyridine, on refroidit le milieu avec un mélange de glace et d'éthanol, on ajoute, goutte à goutte, en 1 heure, 24,6 ml (0,174 mole) d'anhydride trifluoroacétique, on laisse revenir à température ambiante et on poursuit l'agitation pendant 1 heure.
On ajoute de l'eau glacée, on décante, on lave la phase organique sucessivement avec 320 ml de solution 1 N d'acide chlorhydrique, avec de l'eau, avec une solution saturée d'hydrogénocarbonate de sodium, puis une solution saturée de chlorure de sodium, on la sèche sur sulfate de magnésium, et on évapore le solvant. On obtient 40,3 g de produit qu'on utilise tel quel dans l'étape suivante.

7.2. (±) 6-chloro-3,4-dihydro-2*H*-1,4-benzoxazine-3-acétate d'éthyle.

Dans un réacteur de 3 litres, sous agitation magnétique, refroidi à 0°C, on introduit 420 ml d'éthanol, on ajoute lentement, par petites portions, 3,8 g (0,166 mole) de sodium, puis, successivement, et au goutte à goutte, 40 g (0,166 mole) de *N*-(5-chloro-2-hydroxyphényl)trifluoroacétamide et 40 g (0,155 mole) de 4-bromobut-2-énoate d'éthyle pur à 75%, et on chauffe le mélange à 85°C pendant 2 heures.
On évapore le solvant, on reprend le résidu avec 100 ml d'eau et 40 ml de soude 1 N et on l'extrait avec de l'éther diéthylique. On sépare la phase organique, on la lave avec une solution aqueuse saturée de chlorure de sodium, on la sèche sur sulfate de magnésium et on l'évapore. On obtient 28,58 g de produit qu'on purifie par chromatographie sur colonne de gel de silice en éluant avec un mélange cyclohexane/éther isopropylique (50/50).
On obtient 23,72 g de produit.

7.3. (±) 6-chloro-3,4-dihydro-2*H*-1,4-benzoxazine-3-éthanol.

Dans un réacteur de 1 litre on place 150 ml de tétrahydrofurane, on le refroidit avec un mélange de glace et de sel et, sous atmosphère d'argon, on ajoute 5,92 g (0,156 mole) d'hydrure de lithium et d'aluminium puis, goutte à goutte, 23,52 g (0,0973 mole) de (±) 6-chloro-3,4-dihydro-2*H*-1,4-benzoxazine-3-acétate d'éthyle en solution

dans 150 ml de tétrahydrofurane, et on agite le mélange pendant 1,5 heure. On refroidit le réacteur avec un mélange de neige carbonique et d'acétone, et on ajoute, goutte à goutte, 40 ml d'eau et 20 ml de soude 1 N, on agite pendant 0,5 heure, on filtre le précipité sur kieselguhr, on le rince avec du tétrahydrofurane puis avec de l'acétate d'éthyle, et on évapore le solvant. On isole 27,5 g de produit brut qu'on purifie par chromatographie sur colonne de gel de silice en éluant avec un mélange cyclohexane/acétate d'éthyle (50/50).
On obtient 19,67 g de produit.

7.4. (±) 6-chloro-4-[(3-trifluorométhyl)benzoyl]-3,4-dihydro-2*H*-1,4-benzoxazine-3-éthanol.

Dans un ballon de 1 litre on introduit 100 ml de dichlorométhane, 19,17 g (0,09 mole) de (±) 6-chloro-3,4-dihydro-2*H*-1,4-benzoxazine-3-éthanol, 13,3 g (0,096 mole) de carbonate de potassium et on ajoute, goutte à goutte, 20 g (0,096 mole) de chlorure de 3-(trifluorométhyl)benzoyle en solution dans 100 ml de dichlorométhane, et on agite le mélange à température ambiante pendant 3 heures.
On ajoute 90 ml de soude 1 N, on sépare la phase organique, on la lave à l'eau puis avec une solution saturée de chlorure de sodium, on la sèche sur sulfate de magnésium et on évapore le solvant. On obtient 36 g de produit huileux qu'on purifie par chromatographie sur colonne de gel de silice en éluant avec un mélange cyclohexane/acétate d'éthyle (2/1).
On obtient 24,21 g de produit.

7.5.(±) 6-chloro-3-(2-chloroéthyl)-4-[(3-trifluorométhyl)benzoyl]-3,4-dihydro-2*H*-1,4-benzoxazine.

A 24,21 g de (±) 6-chloro-4-[(3-trifluorométhyl)benzoyl]-3,4-dihydro-2*H*-1,4-benzoxazine-3-éthanol en solution dans 260 ml de dichlorométhane on ajoute 18 ml (0,25 mole) de chlorure de thionyle et on agite le mélange à température ambiante pendant 6 heures.
On évapore le solvant, on reprend le résidu avec du toluène et on l'évapore. On purifie les 25,21 g d'huile obtenue par chromatographie sur colonne de gel de silice en éluant avec un mélange cyclohexane/éther isopropylique (50/50).
On obtient 23,73 g de produit.

7.6. (±) 6-chloro-3-[2-(2,3,4,5-tétrahydro-1*H*-3-benzazépin-3-yl)éthyl]-4-[3-(trifluorométhyl)benzoyl]-3,4-dihydro-2*H*-1,4-benzoxazine, oxalate.

A une solution de 2 g (0,005 mole) de (±) 6-chloro-3-(2-chloroéthyl)-4-[(3-trifluorométhyl)benzoyl]-3,4-dihydro-2*H*-benzoxazine dans 20 ml de *N,N*-diméthylformamide, à température ambiante, sous agitation et sous atmosphère d'argon, on ajoute 0,9 g (0,005 mole) de chlorhydrate de 2,3,4,5-tétrahydro-1*H*-3-benzazépine, 1,7 g (0,0124 mole) de carbonate de potassium et 0,82 g (0,005 mole) d'iodure de potassium, et on chauffe le mélange à 110°C pendant 1 heure.
On le refroidit, on ajoute 55 ml d'eau et 50 ml d'éther diéthylique, on sépare les phases et on extrait la phase aqueuse avec deux fois 50 ml d'éther diéthylique. On rassemble les phases organiques, on les lave avec 100 ml de solution saturée de chlorure de sodium, on les sèche sur sulfate de magnésium, on filtre et on évapore le solvant. On obtient 2,5 g de produit sous forme d'huile qu'on purifie par chromatographie sur colonne de gel de silice en éluant avec un mélange dichlorométhane/méthanol (98/2).
On obtient 1,84 g de base pure sous forme d'huile jaune, dont on prépare l'oxalate en ajoutant un équivalent d'acide oxalique et en le recristallisant dans l'éthanol.
Point de fusion : 192-194°C.

Le tableau de la page suivante illustre les structures chimiques et les propriétés physiques de quelques composés selon l'invention.

Légende du tableau

dans la colonne "$R_1$", "n-A-$C_6H_4$" représente un groupe phényle, substitué en position n du cycle, par un groupe A.
dans la colonne "Sel", "-" désigne un composé à l'état de base, "ox." désigne un oxalate et "fum." désigne un fumarate ; lorsque le rapport molaire acide:base est différent de 1:1 il est indiqué entre parenthèses.
dans la colonne "F (°C)", "dec" signifie " fusion avec décomposition".

## Tableau

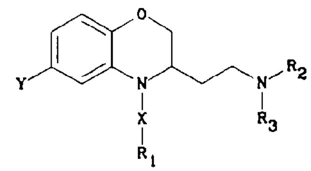

(I)

| N° | $R_1$ | X | Y | $NR_2R_3$ | Sel | F (°C) |
|---|---|---|---|---|---|---|
| 1 | $4-F-C_6H_4-$ | CO | H | 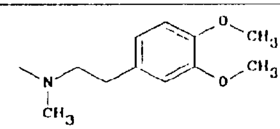 | ox. | 164-166 |
| 2 | $4-CH_3-C_6H_4-$ | $SO_2$ | H | $-N(CH_3)-(CH_2)_3CH_3$ | ox. | 79-81 |
| 3 | $3-CF_3-C_6H_4$ | CO | H | $-N(CH_3)-(CH_2)_3CH_3$ | ox. | 151-152 |
| 4 | $3-CF_3-C_6H_4$ | CO | H | 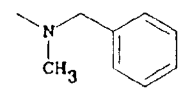 | ox. | 108-110 (dec) |
| 5 | $3-CF_3-C_6H_4$ | CO | H | 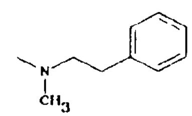 | ox. | 175-176 |
| 6 | $4-CH_3-C_6H_4$ | $SO_2$ | H | 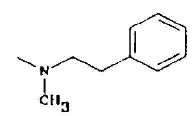 | ox. | 124-126 |
| 7 | $3-CF_3-C_6H_4$ | CO | H | 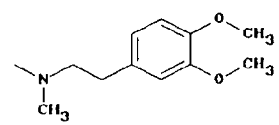 | ox. | 143-144 |

| N° | $R_1$ | X | Y | $NR_2R_3$ | Sel | F (°C) |
|---|---|---|---|---|---|---|
| 8 | $4-CF_3-C_6H_4$ | CO | H | 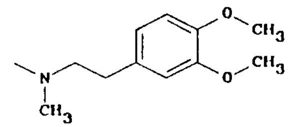 | ox. | 134-136 |
| 9 | $4-CH_3-C_6H_4$ | $SO_2$ | H | 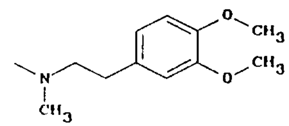 | ox. | 104-106 |
| 10 | $3-CF_3-C_6H_4$ | CO | H | 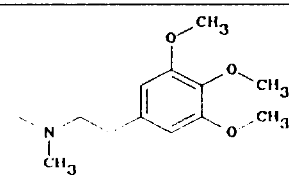 | ox. | 95 (dec) |
| 11 | $3-CF_3-C_6H_4$ | CO | H | 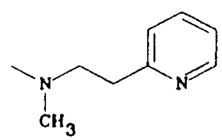 | ox. (2:1) | 105-106 |
| 12 | $4-CH_3-C_6H_4$ | $SO_2$ | H | 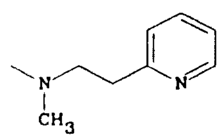 | ox. (2:1) | 148-150 |
| 13 | $4-C_6H_5-C_6H_4$ | CO | H | 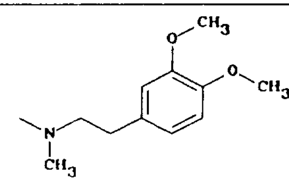 | ox. | 150 |

| N° | $R_1$ | X | Y | $NR_2R_3$ | Sel | F (°C) |
|---|---|---|---|---|---|---|
| 14 | thièn-2-yle | CO | H | 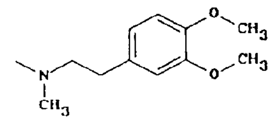 | ox. | 174-176 |
| 15 | $4-CH_3-C_6H_4$ | CO | H | 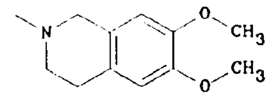 | - | 181-183 |
| 16 | $4-CH_3-C_6H_4$ | $SO_2$ | H | 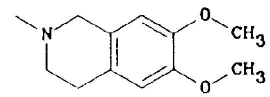 | ox. | 190-192 |
| 17 | $3-CF_3-C_6H_4$ | CO | H | 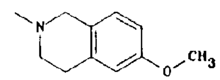 | fum. | 166-168 |
| 18 (±) | $3-CF_3-C_6H_4$ | CO | H | 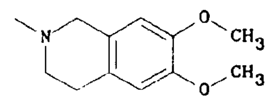 | ox. | 126-128 |
| 18a (+) | $[\alpha]_D^{20}$ = +69° | | | (c = 0,98 ; méthanol) | ox. | 128-129 |
| 18b (-) | $[\alpha]_D^{20}$ = -71° | | | (c = 1,03 ; méthanol) | ox. | 129-130 |

| N° | $R_1$ | X | Y | $NR_2R_3$ | Sel | F (°C) |
|---|---|---|---|---|---|---|
| 19 | $4-CF_3-C_6H_4$ | CO | H | 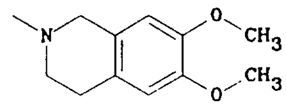 | fum. (1,5:1) | 202-205 |
| 20 | $4-OCH_3-C_6H_4$ | CO | H | 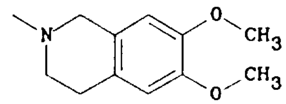 | - | 162 |
| 21 | $4-C_6H_5-C_6H_4$ | CO | H | 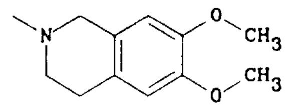 | - | 161-162 |
| 22 | thièn-2-yle | CO | H | 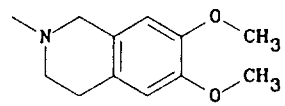 | fum. | 178-180 |
| 23 | $4-CH_3-C_6H_4$ | $SO_2$ | H | 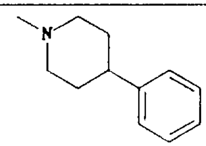 | ox. | 235-237 |
| 24 | $4-CH_3-C_6H_4$ | CO | H | 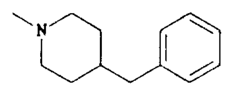 | ox. | 186-188 |
| 25 | $4-CH_3-C_6H_4$ | $SO_2$ | H | 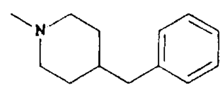 | ox. | 209-211 |

| N° | $R_1$ | X | Y | $NR_2R_3$ | Sel | F (°C) |
|---|---|---|---|---|---|---|
| 26 | $3-CF_3-C_6H_4$ | CO | H | 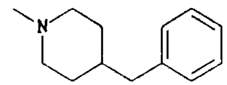 | ox. | 182-183 |
| 27 | $4-C_6H_5-C_6H_4$ | CO | H | 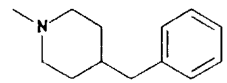 | fum. | 220-223 |
| 28 | $3-CF_3-C_6H_4$ | CO | H | 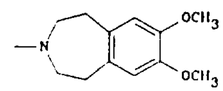 | fum. | 182-184 |
| 29 | $3-CF_3-C_6H_4$ | CO | H | 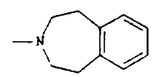 | - | 185-186 |
| 30 | $3-CF_3-C_6H_4$ | CO | H | 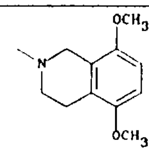 | - | 125-126 |
| 31 | $3-CF_3-C_6H_4$ | CO | F | 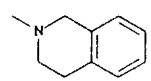 | ox. (1,1:1) | 115-116 |
| 32 | $3-CF_3-C_6H_4$ | CO | F | 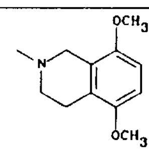 | - | 142-143 |

| N° | $R_1$ | X | Y | $NR_2R_3$ | Sel | F (°C) |
|---|---|---|---|---|---|---|
| 33 | $3-CF_3-C_6H_4$ | CO | F | 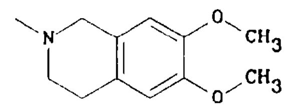 | fum. (0,5:1) | 119-120 |
| 34 | $3-CF_3-C_6H_4$ | CO | Cl | 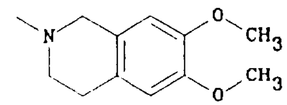 | ox. (1,6:1) | 123-125 |
| 35 | $3-CF_3-C_6H_4$ | CO | Cl | 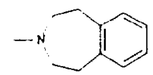 | ox. | 192-194 |
| 36 | $3-CF_3-C_6H_4$ | CO | $CH_3$ | 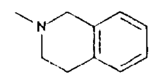 | ox. | 196-198 |
| 37 | $3-CF_3-C_6H_4$ | CO | $CH_3$ | 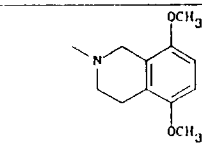 | ox. (0,8:1) | 111-114 |
| 38 | $3-CF_3-C_6H_4$ | CO | $CH_3$ | 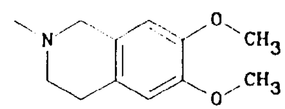 | ox. (0,8:1) | 164-166 |
| 39 | $3-CF_3-C_6H_4$ | CO | $CH_3$ | 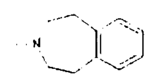 | - | 139-141 |

| N° | $R_1$ | X | Y | $NR_2R_3$ | Sel | F (°C) |
|---|---|---|---|---|---|---|
| 40 | $3-CF_3-C_6H_4$ | CO | $OCH_3$ | 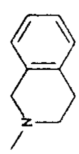 | ox. | 149-151 |
| 41 | $3-CF_3-C_6H_4$ | CO | $OCH_3$ | 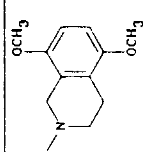 | ox. | 167-169 |
| 42 | $3-CF_3-C_6H_4$ | CO | $OCH_3$ | 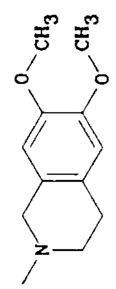 | ox. | 172-174 |

Les composés de l'invention ont fait l'objet d'essais pharmacologiques qui ont montré leur intérêt comme substances actives en thérapeutique.

Inhibition de l'entrée du calcium induite par KCl dans des coupes de cortex de rat immature

On utilise des rats Sprague-Dawley, mâle ou femelle, âgés de 8 jours. Après dislocation cervicale, on excise le cerveau et on prépare des coupes de cortex pariètal.
La concentration intracellulaire en calcium ($[Ca^{2+}]_i$) est mesurée selon la technique décrite dans *J. Pharm. Exp. Ther.* (1992) **261** 324-330. On incube les tranches ainsi prélevées, pendant 75 minutes, à 24°C, dans du tampon Krebs saturé en $O_2/CO_2$ (95%/5%) et contenant du Fura-2 AM™ à la concentration de 7 µM. Après incubation, on rince les coupes plusieurs fois avec ce même tampon et on les laisse dans ce tampon jusqu'à leur utilisation. Pour mesurer la $[Ca^{2+}]_i$, on place les coupes, à 30°C, dans la cuve d'un spectrofluorimètre que l'on perfuse avec du tampon Krebs par l'intermédiaire d'une pompe. On réalise la dépolarisation des tranches en perfusant, pendant 3 minutes, du tampon Krebs contenant du KCl à 50 mM. On introduit le composé à tester dans le liquide de perfusion, 7 minutes après cette première dépolarisation et on réalise une seconde dépolarisation 7 minutes après l'introduction du composé à tester. On suit la fluorescence à deux longueurs d'onde d'excitation, 340 nm (forme liée au calcium) et 380 nm (forme libre), la longueur d'onde d'émission étant 510 nm. On calcule la $[Ca^{2+}]_i$ selon la méthode décrite dans *J. Biol. Chem.* (1985) **260** 3440-3450. On calcule l'effet inhibiteur des composés à tester par rapport à l'augmentation de la $[Ca^{2+}]_i$ induite par du KCl 50 mM prise comme 100%.
Le pourcentage d'inhibition de l'entrée du $Ca^{2+}$, induit par les composés de l'invention, est dose-dépendant et se situe entre 10 et 65% pour des concentrations de 10 à 30 µM.

Ischémie cérébrale complète chez la souris

Les composés de l'invention ont été soumis au test de l'ischémie cérébrale complète chez la souris. L'ischémie est due à un arrêt cardiaque induit par une injection intraveineuse rapide de chlorure de magnésium. Dans ce test on mesure le "temps de survie", c'est-à-dire l'intervalle entre le moment de l'injection de chlorure de magnésium et le dernier mouvement respiratoire observable de chaque souris. Ce dernier mouvement est considéré comme l'indice ultime d'une fonction du système nerveux central.
L'arrêt respiratoire apparaît approximativement 19 secondes après l'injection de chlorure de magnésium.
Des souris mâles (SWISS $OF_1$ IFFA CREDO) sont étudiées par groupes de 10. Elles sont nourries et abreuvées ad libitum avant les essais. Le temps de survie est mesuré 10 minutes après l'administration intrapéritonéale des composés de l'invention. Les résultats sont donnés sous la forme de la différence entre le temps de survie mesuré dans un groupe de 10 souris ayant reçu le composé et le temps de survie mesuré dans un groupe de 10 souris ayant reçu le liquide véhicule. Les rapports entre les modifications dans le terme de survie et la dose du composé sont enregistrés graphiquement selon une courbe semilogarithmique.
Cette courbe permet le calcul de la "dose efficace 3 secondes" ($DE_{3"}$), c'est-à-dire la dose (en mg/kg) qui produit une augmentation de 3 secondes du temps de survie par rapport au groupe témoin de 10 souris non traitées.
Une augmentation de 3 secondes du temps de survie est à la fois significative statistiquement et reproductible.
Les $DE_{3"}$ des composés de l'invention vont de 0,2 à 60 mg/kg par voie intrapéritonéale.

Etude des courants du baryum potentiel-dépendants (" voltage-dépendants") par la technique dite du "patch-clamp".

La mesure des courants du baryum passant par les canaux calciques potentiel-dépendants est effectuée sur des cellules de cortex de rat nouveau-né (Sprague-Dawley) en culture (6 à 10 jours de culture).
Les chambres de mesure, d'un volume de 800 µl, contenant les cellules de cortex de rat, sont placées sur la platine d'un microscope inversé Olympus IMT-2™ et observées au grossissement 400×. Les chambres sont continuellement perfusées (4 à 5 ml/mn) à l'aide d'un dispositif distributeur de solutions acceptant 9 entrées (volume mort<50 µl) dont la sortie unique, constituée d'un tube de polyéthylène de 500 µm d'ouverture, est placée à moins de 3 mm de la cellule étudiée. Ce dispositif a l'avantage de permettre un échange rapide de solution au niveau des cellules étudiées.
La méthode de patch-clamp utilisée est décrite dans *Pfluegers Archives* (1981) **391** 85-100. Un amplificateur Axopatch-1D™ associé à un ordinateur de type AT 386-33MHz, utilisant les logiciels PCLAMP™ de Axon Instruments™ est employé pour la stimulation des cellules, l'acquisition des données et l'analyse des résultats. Pour la réalisation des enregistrements des courants du baryum, des pipettes en verre borosilicaté sont approchées des cellules grâce à un micromanipulateur hydraulique Narishige WR 60™. La pointe des pipettes est remplie de la solution intracellulaire de référence dont la composition (en mM) est la suivante : CsCl (140), $CaCl_2$ (1), $Na_2ATP$ (4), EGTA (11 ; pCa=8), Hepes (10), Tris-OH (pH=7,2). Une fois la configuration de la cellule entière obtenue, la cellule est perfusée avec une solution dite TEA-Baryum dont la composition (en mM) est la suivante : TEA-Cl (144), $BaCl_2$ (5), $MgCl_2$ (2), CsCl (3), glucose (10), Hepes (10), Tris-OH (pH=7,4).
Cette solution permet la mesure du courant calcique (assimilé au courant du baryum passant par les canaux calciques potentiel-dépendants) tout en s'affranchissant des courants sodiques et potassiques.

Le courant du baryum potentiel-dépendant global est obtenu par l'application d'un saut dépolarisant de potentiel d'une durée de 250 ms, portant le potentiel de membrane de -80 mV à -16 mV. La fréquence de stimulation est de 0,25 Hz.

Les composés de l'invention sont mis en solution dans le milieu TEA-baryum et appliqués une fois l'amplitude du courant du baryum stabilisée. Après obtention d'un effet inhibiteur stable, la cellule est de nouveau perfusée avec la solution TEA-baryum de contrôle afin d'observer la réversion de l'effet.

La puissance de l'effet obtenu est comparée à celle d'une solution de cadmium à 100 µM. Les effets bloquants des canaux calciques potentiel-dépendants varient en fonction des doses de composés étudiés et, pour les composés les plus actifs, sont de l'ordre de 66% à la concentration de 1 µM et de 100% à la concentration de 10 µM.

Les résultats des essais effectués sur les composés de l'invention montrent que, *in vitro,* ils ont des propriétés antagonistes calciques neuronales et, *in vivo,* ils ont des propriétés neuroprotectrices et anti-ischémiques.

Ils suggèrent que les composés peuvent être utilisés pour le traitement et la prévention de désordres cérébraux tels que ceux qui sont consécutifs, par exemple, à une attaque ischémique, un arrêt cardiaque ou respiratoire, une thrombose ou une embolie cérébrale, pour le traitement de la sénilité cérébrale, de la démence consécutive aux infarctus multiples, de la démence sénile, par exemple de la maladie de Alzheimer ou de la maladie de Pick, pour le traitement de l'atrophie olivo-ponto-cérébellaire et d'autres maladies neurodégénératives telles que la chorée de Huntington, la sclérose latérale amyotrophique, pour le traitement des traumatismes crâniens ou spinaux, pour la prévention des dommages neuronaux faisant suite à des états convulsifs, pour le traitement de certains cancers, pour le traitement des altérations neurologiques dues au SIDA et pour le traitement des rétinopathies diabétiques.

A cet effet ils peuvent être présentés sous toutes formes pharmaceutiques adaptées à l'administration entérale ou parentérale, en association avec des excipients appropriés, par exemple sous forme de comprimés, dragées, gélules, capsules, suppositoires, solutions ou suspensions buvables ou injectables, dosés pour permettre l'administration journalière de 0,1 à 1000 mg de substance active.

## Revendications

1. Composé, sous la forme d'isomère optique pur ou de mélange d'isomères optiques, répondant à la formule générale (I)

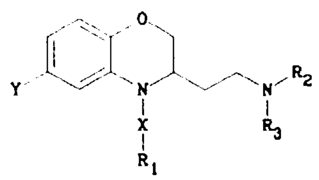

(I)

dans laquelle

Y représente un atome d'hydrogène, de fluor ou de chlore ou un groupe méthyle ou méthoxy,
$R_1$ représente soit un groupe phényle substitué par un atome de fluor ou par un groupe choisi parmi les groupes méthyle, méthoxy, trifluorométhyle et phényle, soit un groupe thién-2-yle,
$R_2$ représente un groupe méthyle,
$R_3$ représente soit un groupe ($C_1$-$C_4$)alkyle, soit un groupe phényl($C_1$-$C_2$)alkyle éventuellement substitué sur le noyau par 2 à 3 groupes méthoxy, soit un groupe 2-(pyridin-2-yl)éthyle, ou bien
$R_2$ et $R_3$ forment, avec l'azote, soit un groupe 4-phényl(pipéridin-1-yle), soit un groupe 4-phénylméthyl(pipéridin-1-yle), soit un groupe 1,2,3,4-tétrahydroisoquinoléin-2-yle, soit un groupe 6-méthoxy-1,2,3,4-tétrahydroisoquinoléin-2-yle, soit un groupe 5,8-diméthoxy-1,2,3,4-tétrahydroisoquinoléin-2-yle, soit un groupe 6,7-diméthoxy-1,2,3,4-tétrahydroisoquinoléin-2-yle, soit un groupe 2,3,4,5-tétrahydro-lH-3-benzazépin-3-yle, soit un groupe 7,8-diméthoxy-2,3,4,5-tétrahydro-1*H*-3-benzazépin-3-yle, et
X représente soit un groupe carbonyle, soit un groupe sulfonyle,

ainsi que ses sels d'addition aux acides pharmaceutiquement acceptables.

2. Composé selon la revendication 1, caractérisé en ce que $R_1$ représente un groupe 3-(trifluorométhyl)phényle,

$R_2$ et $R_3$ forment, avec l'azote, un groupe 6,7-diméthoxy-1,2,3,4-tétrahydroisoquinoléin-2-yle, et
X représente un groupe carbonyle.

3. Procédé de préparation de composés selon la revendication 1, caractérisé en ce que

. lorsque X représente un groupe carbonyle,
on fait réagir un 2-aminophénol de formule générale (II)

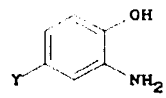

(II)

dans laquelle Y est tel que défini dans la revendication 1, avec de l'anhydride trifluoracétique, pour obtenir l'amide de formule générale (IV)

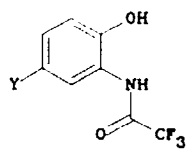

(IV)

que l'on fait réagir avec du 4-bromobut-2-énoate d'éthyle, puis on réduit la fonction ester du 3,4-dihydro-2*H*-1,4-benzoxazine-3-acétate d'éthyle, de formule générale (VI)

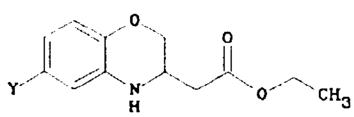

(VI)

pour obtenir le 3,4-dihydro-2*H*-1,4-benzoxazine-3-éthanol de formule générale (VII)

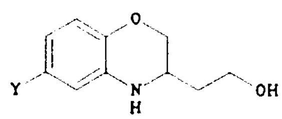

(VII)

que l'on fait réagir un chlorure d'acide de formule générale (VIII)

$$Cl\text{-}CO\text{-}R_1 \qquad (VIII)$$

dans laquelle $R_1$ est tel que défini dans la revendication 1, pour obtenir un alcool de formule générale (IX)

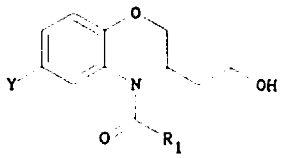

(IX)

que l'on fait réagir avec du chlorure de thionyle pour obtenir un composé de formule générale (X)

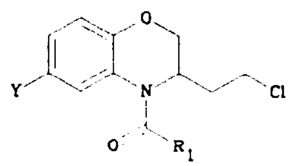

(X)

et, finalement, on fait réagir ce dernier avec une amine de formule générale (XI)

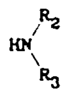

(XI)

dans laquelle $R_2$ et $R_3$ sont tels que définis dans la revendication 1,
ou bien

. lorsque X représente un groupe sulfonyle
on fait réagir un 2-aminophénol de formule générale (II)

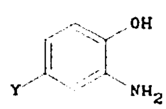

(II)

dans laquelle Y est tel que défini dans la revendication 1, avec un chlorure de formule générale (III')

$$R_1\text{-}SO_2\text{-}Cl \quad (III')$$

dans laquelle $R_1$ est tel que défini dans la revendication 1, pour obtenir un composé de formule générale (IV')

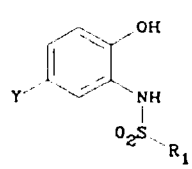

(IV')

que l'on fait réagir avec du 4-bromobut-2-énoate d'éthyle, puis on réduit la fonction ester du composé de formule générale (VI') pour obtenir un composé de formule générale (VII')

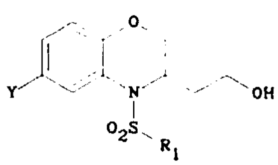

(VII')

que l'on fait réagir avec du chlorure de thionyle, pour obtenir le composé de formule générale (X')

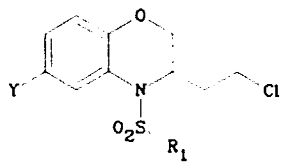

(X')

que l'on condense finalement avec une amine de formule générale (XI)

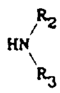

(XI)

dans laquelle $R_2$ et $R_3$ sont tels que définis dans la revendication 1.

4. Procédé selon la revendication 3, caractérisé en ce qu'on isole les énantiomères dextrogyre et lévogyre des alcools de formules générales (IX) et (VII') par voie enzymatique, puis on poursuit la synthèse.

5. Médicament caractérisé en ce qu'il consiste en un composé selon la revendication 1.

6. Composition pharmaceutique caractérisée en ce qu'elle contient un composé selon la revendication 1 en association avec tout excipient approprié.

7. Composés répondant à la formule générale (XII)

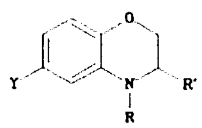

(XII)

dans laquelle

Y est tel que défini dans la revendication 1,
soit R représente un atome d'hydrogène et R' un groupe 2-hydroxyéthyle,
soit R représente un groupe $-COR_1$ où $R_1$ est un groupe phényle substitué par un atome de fluor ou par un groupe choisi parmi les groupes méthyle, méthoxy, trifluorométhyle et phényle, ou un groupe thièn-2-yle et R' représente un groupe 2-hydroxyéthyle ou un groupe 2-chloroéthyle,
soit R représente un groupe $-SO_2R_1$ où $R_1$ est un groupe phényle substitué par un atome de fluor ou par un groupe choisi parmi les groupes méthyle, méthoxy, trifluorométhyle et phényle, ou un groupe thièn-2-yle et R' représente un groupe 2-hydroxyéthyle ou un groupe 2-chloroéthyle ou un groupe éthoxycarbonylméthyle,

utiles pour la synthèse de composés selon la revendication 1.

**Patentansprüche**

1. Verbindung in Form des reinen optischen Isomeren oder einer Mischung von optischen Isomeren, der allgemeinen Formel (I)

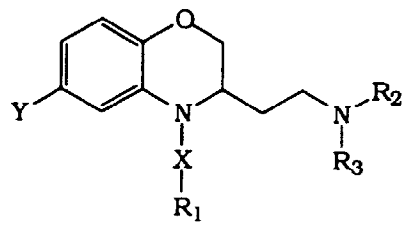

(I)

in der

Y ein Wasserstoffatom. ein Fluoratom, ein Chloratom, eine Methylgruppe oder eine Methoxygruppe,
$R_1$ entweder eine Phenylgruppe, die durch ein Fluoratom oder eine aus Methyl-, Methoxy-, Trifluormethyl- und Phenylgruppen ausgewählte Gruppe substituiert ist, oder eine Thien-2-yl-gruppe,
$R_2$ eine Methylgruppe,
$R_3$ entweder eine ($C_1$-$C_4$)-Alkylgruppe oder eine Phenyl-($C_1$-$C_2$)-alkylgruppe, die gegebenenfalls am Kern durch 2 bis 3 Methoxygruppen substituiert ist, oder eine 2-(Pyridin-2-yl)-ethylgruppe, oder
$R_2$ und $R_3$ zusammen mit dem Stickstoffatom entweder eine 4-Phenyl-(piperidin1-yl)-gruppe, oder eine 4-Phenylmethyl-(piperidin-1-yl)-gruppe, oder eine 1,2,3,4-Tetrahydroisochinolin-2-yl-gruppe, oder eine 6-Methoxy-1,2,3,4-tetrahydroisochinolin-2-yl-gruppe, oder eine 5.8-Dimethoxy-1,2,3,4-tetrahydroisochinolin-2-yl-gruppe, oder eine 6,7-Dimethoxy-1,2,3,4-tetrahydroisochinolin-2-yl-gruppe, oder eine 2,3,4,5-Tetrahydro-1*H*-3-benzazepin-3-yl-gruppe, oder eine 7,8-Dimethoxy-2,3,4,5-tetrahydro-1*H*-3-benzazepin-3-yl-gruppe und
X entweder eine Carbonylgruppe oder eine Sulfonylgruppe bedeuten, sowie deren Additionssalze mit pharmazeutisch annehmbaren Säuren.

**2.** Verbindung nach Anspruch 1, dadurch gekennzeichnet, daß $R_1$ eine 3-(Trifluormethyl)-phenylgruppe darstellt.

$R_2$ und $R_3$ zusammen mit dem Stickstoffatom eine 6,7-Dimethoxy-1,2,3,4-tetrahydroisochinolin-2-yl-gruppe bilden und
X eine Carbonylgruppe darstellt.

**3.** Verfahren zur Herstellung der Verbindungen nach Anspruch 1, dadurch gekennzeichnet, daß man

- wenn X eine Carbonylgruppe darstellt.
ein 2-Aminophenol der allgemeinen Formel (II)

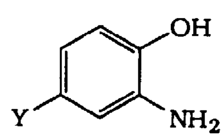

(II)

in der Y die in Anspruch 1 angegebenen Bedeutungen besitzt, mit Trifluoressigsäureanhydrid umsetzt zur Bildung des Amids der allgemeinen Formel (IV)

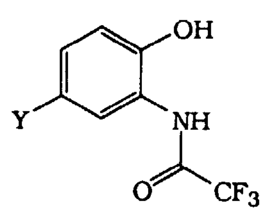

(IV)

welches man mit 4-Brombut-2-ensäureetehylester umsetzt, wonach man die Esterfunktion des 3,4-Dihydro-2*H*-1,4-benzoxazin-3-essigsäureethylesters der allgemeinen Formel (VI)

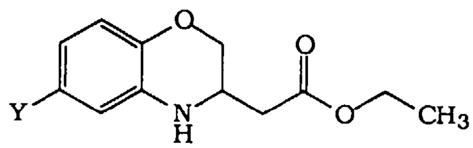

(VI)

reduziert zur Bildung des 3,4-Dihydro-2*H*-1,4-benzoxazin-3-ethanols der allgemeinen Formel (VII)

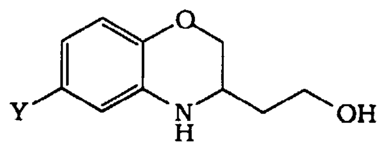

(VII)

welches man mit einem Säurechlorid der allgemeinen Formel (VIII)

$$Cl\text{-}CO\text{-}R_1 \qquad (VIII)$$

in der $R_1$ die in Anspruch 1 angegebenen Bedeutungen besitzt, umsetzt zur Bildung eines Alkohols der allgemeinen Formel (IX)

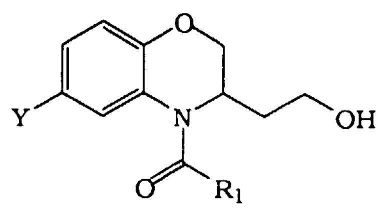

(IX)

welchen man mit Thionylchlorid umsetzt zur Bildung einer Verbindung der allgemeinen Formel (X)

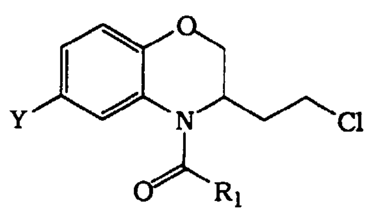

(X)

wonach man schließlich diese letztere Verbindung mit einem Amin der allgemeinen Formel (XI)

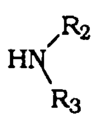

(XI)

in der $R_2$ und $R_3$ die in Anspruch 1 angegebenen Bedeutungen besitzen, umsetzt, oder

- wenn X eine Sulfonylgruppe darstellt.
man ein 2-Aminophenol der allgemeinen Formel (II)

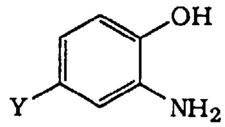

(II)

in der Y die in Anspruch 1 angegebenen Bedeutungen besitzt, mit einem Chlorid der allgemeinen Formel (III')

$$R_1\text{-}SO_2\text{-}Cl \qquad (III')$$

in der $R_1$ die in Anspruch 1 angegebenen Bedeutungen besitzt, umsetzt zur Bildung einer Verbindung der allgemeinen Formel (IV')

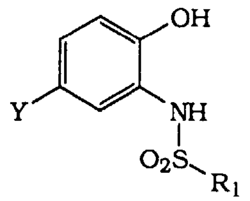

(IV')

welche man mit 4-Brombut-2-ensäureethylester umsetzt, wonach man die Esterfunktion der Verbindung der allgemeinen Formel (VI') reduziert zur Bildung einer Verbindung der allgemeinen Formel (VII')

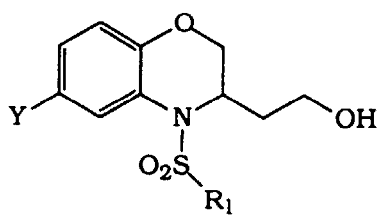

(VII')

welche man mit Thionylchlorid umsetzt zur Bildung der Verbindung der allgemeinen Formel (X')

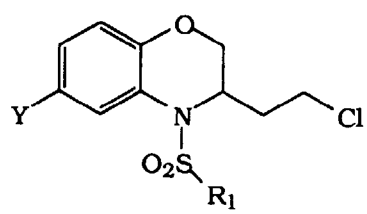

(X')

welche man schließlich mit einem Amin der allgemeinen Formel (XI)

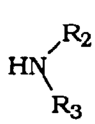

(XI)

in der $R_2$ und $R_3$ die in Anspruch 1 angegebenen Bedeutungen besitzen, kondensiert.

**4.** Verfahren nach Anspruch 3, dadurch gekennzeichnet, daß man die rechtsdrehenden und linksdrehenden Enantiomeren der Alkohole der allgemeinen Formeln (IX) und (VII') auf enzymatischem Wege trennt und dann die Synthese fortsetzt.

5. Arzneimittel, dadurch gekennzeichnet, daß es aus einer Verbindung nach Anspruch 1 besteht.

6. Pharmazeutische Zubereitung, dadurch gekennzeichnet, daß sie eine Verbindung nach Anspruch 1 in Kombination mit irgendeinem geeigneten Trägermaterial enthält.

7. Verbindungen der allgemeinen Formel (XII)

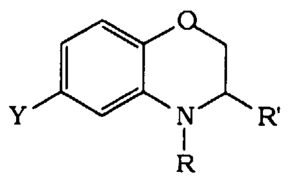

(XII)

in der

Y die in Anspruch 1 angegebenen Bedeutungen besitzt,
entweder R ein Wasserstoffatom und R' eine 2-Hydroxyethylgruppe bedeuten, oder R eine Gruppe $-COR_1$, in der $R_1$ eine durch ein Fluoratom oder durch eine Gruppe ausgewählt aus Methyl-. Methoxy-. Trifluormethyl- und Phenylgruppen substituierte Phenylgruppe oder eine Thien-2-yl-gruppe darstellt und R' eine 2-Hydroxyethyl- oder eine 2-Chlorethylgruppe bedeutet,
oder R eine Gruppe $-SO_2R_1$, in der $R_1$ eine Phenylgruppe, die durch ein Fluoratom oder eine aus Methyl-, Methoxy-, Trifluormethyl- und Phenylgruppen ausgewählte Gruppe substituiert ist, oder eine Thien-2-yl-gruppe darstellt, und R' eine 2-Hydroxyethylgruppe oder eine 2-Chlorethylgruppe oder eine Ethoxycarbonylmethylgruppe bedeutet,

welche für die Synthese der Verbindungen nach Anspruch 1 nützlich sind.

## Claims

1. Compound, in the form of the pure optical isomer or a mixture of optical isomers, corresponding to the general formula (I)

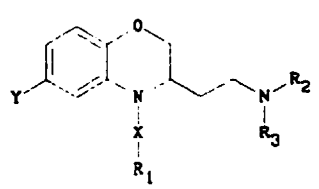

(1)

in which

Y represents an atom of hydrogen, fluorine or chlorine or a methyl or methoxy group,
$R_1$ either represents a phenyl group which is substituted with a fluorine atom or with a group chosen from among the groups methyl, methoxy, trifluoromethyl and phenyl, or represents a 2-thienyl group,
$R_2$ represents a methyl group,
$R_3$ either represents a $(C_1-C_4)$-alkyl group, or represents a phenyl- $(C_1-C_2)$-alkyl group which is optionally substituted on the ring by 2 to 3 methoxy groups, or represents a 2-(2-pyridyl)ethyl group, or else
$R_2$ and $R_3$ form, with the nitrogen, either a 4-phenyl(1-piperidyl) group or a 4-phenylmethyl(1-piperidyl) group or a 1,2,3,4-tetrahydro-2-isoquinolyl group or a 6-methoxy-1,2,3,4-tetrahydro-2-isoquinolyl group or a 5,8-dimethoxy-1,2,3,4-tetrahydro-2-isoquinolyl group or a 6,7-dimethoxy-1,2,3,4-tetrahydro-2-isoquinolyl group or a 2,3,4,5-tetrahydro-1H-3-benzazepin-3-yl group or a 7,8-dimethoxy-2,3,4,5-tetrahydro-1H-3-benzazepin-3-yl group, and
X either represents a carbonyl group or represents a sulphonyl group,

and its addition salts with pharmaceutically acceptable acids.

**2.** Compound according to Claim 1, characterized in that $R_1$ represents a 3-(trifluoromethyl)phenyl group,

$R_2$ and $R_3$ form, with the nitrogen, a 6,7-dimethoxy-1,2,3,4-tetrahydro-2-isoquinolyl group, and
X represents a carbonyl group.

**3.** Process for the preparation of compounds according to Claim 1, characterized in that

- when X represents a carbonyl group
a 2-aminophenol of general formula (II)

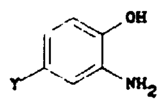

(II)

in which Y is as defined in Claim 1 is reacted with trifluoroacetic anhydride in order to obtain the amide of general formula (IV)

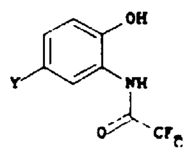

(IV)

which is reacted with ethyl 4-bromobut-2-enoate, then the ester functional group of ethyl 3,4-dihydro-2H-1,4-benzoxazine-3-acetate, of general formula (VI)

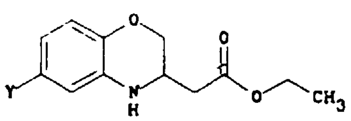

(VI)

is reduced in order to obtain the 3,4-dihydro-2H-1,4-benzoxazine-3-ethanol of general formula (VII)

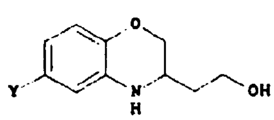

(VII)

which is reacted with an acid chloride of general formula (VIII)

C1-CO-$R_1$ (VIII)

in which $R_1$ is as defined in Claim 1 in order to obtain an alcohol of general formula (IX)

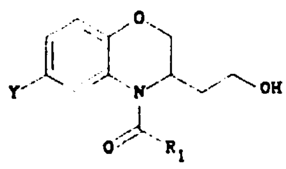

(IX)

which is reacted with thionyl chloride in order to obtain a compound of general formula (X)

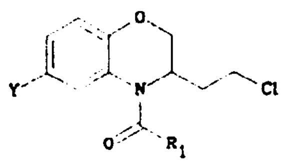

(X)

and, finally, the latter compound is reacted with an amine of general formula (XI)

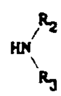

(XI)

in which $R_2$ and $R_3$ are as defined in Claim 1,
or else
- when X represents a sulphonyl group a 2-aminophenol of general formula (II)

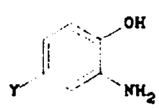

(II)

in which Y is as defined in Claim 1 is reacted with a chloride of general formula (III')

$$R_1\text{-}SO_2\text{-}C1 \qquad \text{(III')}$$

in which $R_1$ is as defined in Claim 1 in order to obtain a compound of general formula (IV')

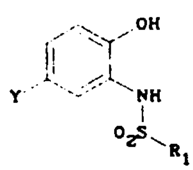

(IV')

which is reacted with ethyl 4-bromobut-2-enoate, then the ester functional group of the compound of general formula (VI') is reduced in order to obtain a compound of general formula (VII')

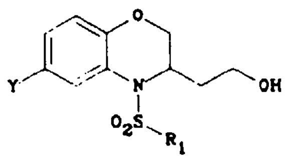

(VII')

which is reacted with thionyl chloride in order to obtain the compound of general formula (X')

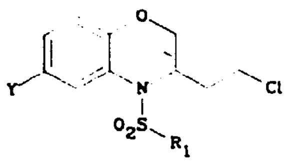

(X')

which is finally condensed with an amine of general formula (XI)

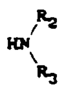

(XI)

in which $R_2$ and $R_3$ are as defined in Claim 1.

**4.** Process according to Claim 3, characterized in that the dextrorotatory and laevorotatory enantiomers of the alcohols of general formulae (IX) and (VII') are isolated by an enzymatic route before the synthesis is continued.

**5.** Medicament, characterized in that it consists of a compound according to Claim 1.

**6.** Pharmaceutical composition, characterized in that it contains a compound according to Claim 1 in combination with any suitable excipient.

**7.** Compounds corresponding to the general formula (XII)

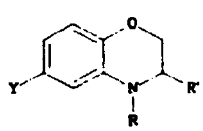

(XII).

in which

Y is as defined in Claim 1,
R either represents a hydrogen atom and R' represents a 2-hydroxyethyl group,
or R represents a group $-COR_1$ in which $R_1$ is a phenyl group which is substituted by a fluorine atom or by a group chosen from among the methyl, methoxy, trifluoromethyl and phenyl groups, or is a 2-thienyl group, and R' represents a 2-hydroxyethyl group or a 2-chloroethyl group,
or R represents a group $SO_2R_1$ in which $R_1$ is a phenyl group which is substituted by a fluorine atom or by a group chosen from among the methyl, methoxy, trifluoromethyl and phenyl groups, or is a 2-thienyl group, and R' represents a 2-hydroxyethyl group or a 2-chloroethyl group or an ethoxycarbonylmethyl group,

which can be used for the synthesis of compounds according to Claim 1.